# EUROPEAN PATENT APPLICATION

(11) **EP 1 122 308 A1**
(43) Date of publication of application: **08.08.2001**
(21) Application number: 99947899.3
(22) Date of filing: 14.10.1999
(51) Int. Cl.: C12N 15/00, C07K 14/00

(54) **METHOD FOR AMPLIFYING FOREIGN GENE**

(30) Priority: 15.10.1998 JP 29269798
(71) Applicant: KYOWA HAKKO KOGYO CO., LTD., Chiyoda-ku, Tokyo 100-8185 (JP); Horiuchi, Takashi, Okazaki-shi, Aichi 444-0840 (JP)
(72) Inventor: HORIUCHI, Takashi, Okazaki-shi, Aichi 444-0840 (JP); KOBAYASHI, Takehiko, Okazaki-shi, Aichi 444-0874 (JP)
(74) Representative: VOSSIUS & PARTNER
(86) International application number: JP9905673
(87) International publication number: WO0022107

(57) **Abstract**

The present invention relates to a method for amplifying a foreign gene by arranging a recombination hot spot region and an autonomously replicating sequence close to a foreign gene and expressing a protein having an activity to block replication fork progression.

## Description

### Technical Field

The present invention relates to a method for amplifying a foreign gene, and to a method for producing a protein encoded by that foreign gene using the foreign gene amplified by that method.

### Background Art

Such phenomena as amplification of oncogene like c-myc, N-myc, and so on accompanied by neoplastic proliferation [Nature, 299, 61, (1982); Cell, 35, 359 (1983); Nature, 305, 245, (1983)], and increase of copy numbers of a dihydrofolate reductase (dhfr) gene in the presence of a folate analogue, methotrexate (MTX), which depends on the concentration of MTX are known as phenomena of gene amplification in eukaryotic cells [Cold Spring Harbor Symp. Quant. Bol. XLII, 649 (1978)].

MTX-dhfr system, generally used as a method for amplifying genes in animal host cells, utilizes the phenomena described above. The MTX-dhfr system, however, has numbers of disadvantages: after screening gene-transferred cells, it takes time to breed and screen gene-amplified cells due to gradually increasing the concentration of MTX; inconstant effectiveness of gene amplification due to the existence of cells capable of growing in the presence of high concentration of MTX not by the amplification of the introduced gene, but as an improved excretion ability of MTX, inactivation ability of MTX, and such among cells; and limitation of cells which are applicable as host, due to the difference in sensitivity to MTX among cells; etc. In addition, use of oncogenes, such as c-myc, for gene amplification are thought to be undesirable in cell culture systems for the production of substances in terms of safety.

A ribosomal RNA (abbreviated to rRNA, hereinafter) gene has a repetitive structure of a unit composed of an rRNA gene and a spacer sequence in eukaryotic cell, such as yeast, pea, Xenopus laevis, rat, mice, and human, and prokaryotic cells, such as E. coli, [Annu. Rev. Biochem., 49, 727, (1980); Nuc. Acids Res., 22, 5038 (1994); EMBO J., 7, 303 (1988) ; Mol. Cell. Biol., 13, 6600 (1993) ; J. Biol. Chem., 271, 2608 (1996); Chromosoma, 104, 511, (1996); DNA replication in Eukaryotic cell, Cold Spring Harbor Laboratory Press, (1996); J. Bacteriol., 177, 783 (1995)]. It is proposed that a general mechanism regulating the copy number of the rRNA genes during amplification of the rRNA gene exists. Moreover, various amplification phenomena below have been found. In the mutant of Neurospora crassa and Drosophila in which the repetitive structure of the rRNA gene has been partially deleted, it is found that the copy numbers recover to its original copy number with proliferation [Proc. Natl. Acad. Sci. USA, 60, 509 (1968); Chromosoma, 93, 337 (1986)]. It is known that the copy number of the rRNA gene in the maturation process of eggs in Xenopus laevis increases 1500 times from its original 450 copies [Science, 160, 272 (1986)]. Moreover, besides the rRNA gene, the copy number of actin genes in chickens has been found to increase in the early stage of myogenesis [Gene Amplification, Cold Spring Harbor Laboratory Press (1982)]. Such phenomena of gene amplification during development, growth and regeneration of normal organisms have been found, but the mechanism of it is not revealed well.

As a biological phenomenon, in the above-mentioned gene amplification, part being amplified is very long. Therefore, it is suggested that the gene unit amplified needs to contain a replication origin. In fact, replication origins were identified in dhfr and c-myc genes, which are known as amplifying genes [DNA replication in Eukaryotic Cells, Cold Spring Harbor Laboratory Press (1996)]. Furthermore a large quantity of gene amplification on chromosomes presumably requires efficient recombination of genes, and also a DNA recombination hot spot on chromosomes.

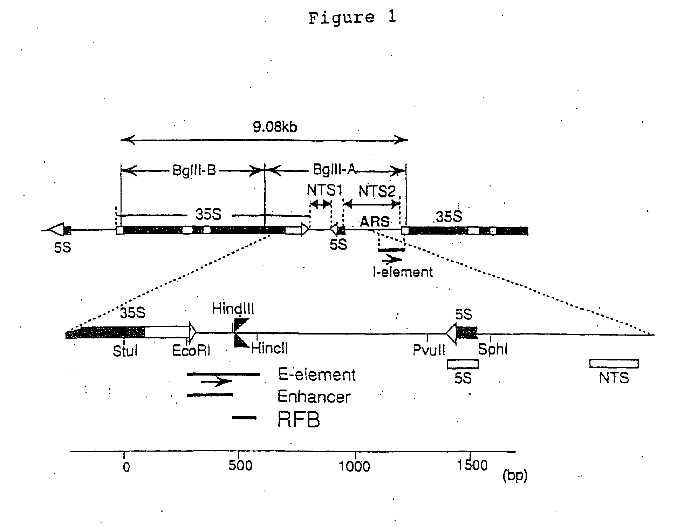

In yeast (Saccharomyces cerevisiae), the rRNA gene exists as a tandem repeated structure of about 150 copies on chromosome XII [Proc. Natl. Acad. Sci. USA, 76, 410 (1979)]. A single repetitive unit is composed of, as shown in Figure 1, two rRNA genes (35S and .5S) and two non-transcribed spacer regions (NTS1 and NTS2) . 35S rRNA gene is specifically transcribed by RNA polymerase I and 5S rRNA by RNA polymerase III. Autonomously replicating sequence (ARS) comprising a replication origin exists in NTS2 [Nucl. Acids Res. 12, 2955 (1988); Mol. Cell. Biol., 8, 4927 (1988)]. A BglII fragment in a repetitive unit of the rRNA gene has the activity to stimulate homologous recombination of its flanking genes, and is thought to be a recombination hot spot. This fragment site is designated HOT1 [Cell, 39, 377 (1984)].

HOT 1 comprises two regions necessary for the transcription of the 35S rRNA; a region comprising an enhancer in NTS1, called E-element [Cell, 39, 663 (1984); Mol. Cell. Biol., 13, 1283, (1993)], and a region comprising the transcription initiation site of the 35S rRNA gene in NTS2, called I-element [Cell, 48, 1071 (1987)]. RNApolymerase I has been reported to be essential for the activity of HOT1 to enhance frequency of homologous recombination of its flanking genes [Genetics, 141, 845 (1995)].

The repetitive unit of the rRNA gene has a replication fork blocking (RFB) region which inhibits replication fork progression from the replication origins in only one direction (the replication fork progressing to left from the right ARS in Figure 1) [Mol. Gen. Genet., 233, 355 (1992) ; Cell, 71, 267, (1992)]. RFB region is composed of a specific sequence of about 100 residues, existing in the region overlapping with E-element in NTS1 [Mol. Cell. Biol., 8, 4927 (1988) ; Cell, 55, 637 (1988); Mol. Gen. Genet., 233, 355 (1992); Cell, 71, 267 (1992)]. Therefore, in order to examine relation between the replication fork blocking and the activity of HOT1 to enhance the frequency of homologous recombination of its flanking genes, yeast mutants which had defect in the activity of HOT1 to enhance the frequency of homologous recombination of its flanking genes were screened, and a strain fob1-4 was discovered in which both replication fork blocking activity at the RFB site and activity of HOT1 to enhance the frequency of homologous recombination of its flanking genes were inactivated.

Using strain fob1-4, FOB1 gene was obtained as a gene capable of complementing both defective activities in this strain.

FOB1 gene is a gene existing on chromosome IV, encoding a protein composed of 566 amino acids shown in SEQ ID NO: 25, which is not essential for the growth. No gene having a homology to FOB1 gene has been identified in the DNA data base so far [Genes to Cells, 1, 465 (1996)].

Working mechanism responsible for the above activities of the FOB1 gene has not been revealed.

### Disclosure of the Invention

In order to improve productivity of proteins encoded by recombinant genes, a method for recombinant gene amplification which enables stably maintaining the amplified gene and safely and reliably amplifying numerous copies of the introduced gene is required. Amethod for recombinant gene amplification which makes it possible to rapidly screen cells with improved productivity of recombinant protein by gene amplification is needed.

The inventors focused on the fact that a large number of copies of rRNA gene existed as a repetitive structure and enthusiastically examined the possibility of amplifying a target gene by clarifying the mechanism of rRNA gene amplification and by using this mechanism.

Specifically, the mechanism related to gene amplification was revealed by the following method.

In the present invention, to clarify the mechanism of rRNA gene amplification, the inventors focused on the relation between the replication fork blocking (RFB) region, which is proposed to be involved in gene replication in yeast, and the FOB1 gene.

The blocking mechanism to the replication fork during gene replication was analyzed by comparing gene amplification of a mutant in which related genes were disrupted to that of a wild-type strain with identical genotypes except for the related genes.

It is thought that genetic recombination efficiently occurs during gene amplification. Therefore, present inventors focused on the DNA recombination hot spot on chromosomes. HOT1, a region in the rRNA gene having the activity to enhance the frequency of homologous recombination of its flanking genes, requires RNA polymerase I. Accordingly, amplification of the rRNA gene in an RNA polymerase I-defective mutant was compared to that of a wild-type strain.

Moreover, it is known that both replication fork blocking activity at the RFB site and the activity of a HOT1 fragment to enhance gene recombination disappear by the mutation of the FOB1 gene in yeast. Therefore, we prepared an RNA polymerase I-defective mutant in which the FOB1 gene was also disrupted.

As an RNA polymerase I-defective mutant, a strain NOY408-1a, in which RPA135 gene encoding A135, the second largest subunit of RNA polymerase I, is disrupted [Proc. Natl. Acad. Sci. USA, 88, 3962 (1991), genotype: MATα, ade2-1 ura3-1 his3-11 trp2-3, 112 canl-100 rpa135:: LEU2 pNDY102], was used.

For comparison to the defective mutant, a diploid strain NOY408 [Mol. Cell. Biol., 11, 754 (1991); Proc. Natl. Acad. Sci. USA, 88, 3962 (1991), genotype: MATa/MATα ade2-1/ade2-1 ura3-1/ura3-1 his3-11/his3-11 trp1-1/trp1-1 leu2-3, 112/leu2-3, 112 can1-100/can1-100 rpa135::LEU2/RPA135 pNDY102], and a haploid strain NOY408-1b, [Proc. Natl. Acad. Sci. USA, 88, 3962 (1991), genotype: MATa ade2-1 ura3-1 his3-11 trp2-3, 112 can1-100 pNOY102] were used as wild-type strains.

As a mutant defective in RNA polymerase I and FOB1 gene, strain NOY408-1af, in which the FOB1 gene of strain NOY408-1a above was disrupted, was used.

The copy numbers of the rRNA gene on chromosome XII in each defective mutant and their wild-type strain were compared and quantified using Southern blot method [J. Mol. Biol., 98, 503 (1975)], and competitive PCR [Proc. Natl. Acad. Sci. USA, 87, 2725 (1990)].

Result obtained by quantifying the copy number of a gene whose copy number was known to be 1 (a reference gene) by the same method as above was used as an internal standard for more precise quantification. Moreover, the exact copy number was calculated by competitive PCR.

The results were as follows: 1) in yeast, the copy number of the rRNA gene in mutant strains defective in RNA polymerase I which is necessary for the activity of recombination hot spot region HOT1 to enhance homologous recombination was decreased to one half; 2) the copy number of the rRNA gene in mutant strains defective in the RNA polymerase I and the FOB1 gene was more decreased than the RNA polymerase I-defective mutant; 3) the copy number of the rRNA gene in mutant strains defective in RNA polymerase I and the FOB1 gene was not recovered at all by an overexpression of RNA polymerase I gene alone, but was recovered by expressing RNA polymerase I and FOB1 gene together; 4) amplification of the rRNA gene requires the recombination hot spot region, autonomously replicating sequence, and an expressed protein of the FOB1 gene. The present invention was accomplished by elucidating the general mechanism which regulates the copy number of the rRNA gene.

Specifically, the present invention relates to the following (1) to (20).
(1) A method for amplifying a foreign gene, the method comprising arranging a recombination hot spot region and an autonomously replicating sequence close to a foreign gene and expressing a protein having an activity to block replication fork progression.
(2) The method of (1), wherein the recombination hot spot region and the autonomously replicating sequence is arranged upstream and/or downstream of the foreign gene.
(3) The method of (1), wherein the recombination hot spot region is derived from HOT1.
(4) The method of (1) or (2), wherein the recombination hot spot region comprises a replication fork blocking (RFB) region.
(5) The method of (1), wherein the recombination hot spot region comprises a DNA replication terminus (Ter).
(6) The method of (1), wherein the protein having an activity to block replication fork progression is a protein comprising the amino acid sequence of SEQ ID NO: 25 or 26, or a protein comprising the amino acid sequence in which one or several amino acids are deleted, replaced, or added and having the activity to block replication fork progression.
(7) A protein comprising the amino acid sequence of SEQ ID NO: 25 or 26 in which one or several amino acids are deleted, replaced, or added and having an activity to block replication fork progression.
   Deletion, replacement or addition of amino acids described above can be performed by site-directed mutagenesis, which is a well known prior art to the application. One or several amino acids mean as many as amino acids which can be deleted, replaced, or added by site-directed mutagenesis.
   A protein comprising the amino acid sequence of SEQ ID NO: 25 or 26 in which one or several amino acids are deleted, replaced, or added and having an activity to block replication fork progression can be prepared according to the methods described in Molecular Cloning, A laboratory Manual, Second Edition, Cold Spring Harbor Laboratory Press (1989) (abbreviated to Molecular Cloning, Second Edition, hereinafter) ; Current Protocols in Molecular Biology, Supplement 1-38, John Wiley & Sons (1987-1997) (abbreviated to Current Protocol in Molecular Biology, hereinafter); Nucleic Acids Research, 10, 6487 (1982); Proc Natl. Acad. Sci. USA, 79, 6409 (1982); Gene, 34, 315 (1985); Nucleic Acids Research, 13, 4431 (1985); Proc Natl. Acad. Sci. USA, 82, 488 (1985); Proc Natl. Acad. Sci. USA, 81, 5662 (1984); Science, 224, 1431 (1984); PCT WO85/00817 (1985); Nature, 316, 601 (1985); etc.
(8) A DNA encoding the protein of (7).
(9) A DNA hybridizing to the DNA of (8) under stringent conditions and encoding a protein having an activity to block replication fork progression.
(10) A DNA hybridizing to the DNA of SEQ ID NO: 27 or 28 under stringent conditions and encoding a protein having an activity to block replication fork progression.
   The term "a DNA hybridizing under stringent conditions and encoding a protein having an activity to block replication fork progression" above means a DNA obtained by utilizing, for example, colony hybridization technique, plaque hybridization technique, or Southern hybridization technique, with the DNA of (8), (9) or (10) above as a probe. Specifically it is a DNA identified by hybridizing at 65°C in the presence of 0.7 to 1.0 M NaCl with a filter on which a DNA derived from a colony or a plaque is fixed, and washing the filter in 0.1 to 2x saline-sodium citrate (SSC) solution (1x SSC solution is composed of 150 mM sodium chloride, 15 mM sodium citrate) at 65°C.
   Hybridization can be performed according to the methods described, for example, in Molecular Cloning, Second Edition, Current Protocol in Molecular Biology [DNA Cloning 1: Core Techniques, A Practical Approach, Second Edition, Oxford University Press (1995)].
   Specifically, a hybridizable DNA is a DNA having at least 80% or higher homology, or preferably 95% or higher homology to the nucleotide sequence of SEQ ID NO: 27 or 28.
(11) A recombinant vector obtained by inserting the DNA of any one of (8) to (10) into a vector and capable of expressing a protein encoded by the DNA.
(12) An amplification vector for a foreign gene, the vector obtained by arranging, in an expression vector for a foreign gene, a recombination hot spot region and an autonomously replicating sequence close to a foreign gene.
(13) The amplification vector of (12), wherein the recombination hot spot region and the autonomously replicating sequence is arranged upstream and/or downstream of the foreign gene.
(14) The amplification vector of (12), wherein the recombination hot spot region is derived from HOT1.
(15) The amplification vector of (12) or (13), wherein the recombination hot spot region comprises a replication fork blocking (RFB) region.
(16) The amplification vector of (12), wherein the recombination hot spot region comprises a DNA replication terminus (Ter).
(17) The amplification vector of any one of (12) to (16), wherein the vector comprises the DNA of any one of (8) to (10).
(18) A transformant obtained by introducing vectors of any one or more of (12) to (17) into a host cell.
(19) A method for amplifying a foreign gene, the method comprising using the transformant of (18).
(20) A method for producing a protein encoded by a foreign gene, the method comprising using the transformant of (18).

The present invention is described in detail below. A foreign gene can be amplified by arranging a recombination hot spot region and an autonomously replicating sequence close to the foreign gene and expressing a protein having an activity to block replication fork progression.

Any gene can be used as a foreign gene. A recombination hot spot region is a region which remarkably enhances homologous recombination of its franking genes during gene replication. In the present invention, any region that can markedly improve the homologous gene recombination can be used.

Specifically, examples are HOT1 of yeast [Cell, 39, 377 (1984)], and HOT A, HOT B, and HOT C of E. coli. Whole region of a hot spot is not always necessary, and solely an important region within the hot spot region, for example the replication fork blocking (RFB) region within a hot spot region, can be used. Specifically, for example, E-element region existing in HOT1 of yeast [Cell, 39, 663 (1984); Mol. Cell. Biol., 13, 1283, (1993)], and TerA, TerB, and TerC existing within HOT A, HOT B, and HOT C of E. coli can be used. A region necessary for the activity to enhance the frequency of homologous recombination within the hot spot region, for example, I-element region existing within HOT1 of yeast, can be used [Cell, 48, 1071 (1987)].

The RFB herein is a region in which the replication fork progression from a replication origin during gene replication is arrested only in one direction.

An autonomously replicating sequence (ARS) is a region which comprises a replication origin and which makes replication of a gene from the replication origin possible. Any region can be used as long as it is able to replicate. Specifically, for example, ARS found within the rRNA gene of yeast can be used.

The recombination hot spot region and the autonomously replicating sequence can be arranged upstream or downstream of a foreign gene as long as they are arranged close to the foreign gene. Moreover; they can be arranged both upstream and downstream of the foreign gene.

A region containing the recombination hot spot region, the autonomously replicating sequence, and the foreign gene arranged in such a manner is inserted into a chromosome or into a vector, and the foreign gene is amplified in the presence of a protein having an activity to block replication fork progression.

As a protein having an activity to block replication fork progression, any protein with that activity can be used. Specifically, for example, a protein encoded by FOB1 gene found in yeast, and a protein encoded by tau (tus) gene found in E. coli [EMBO J., 8, 2435 (1989) ; Proc Natl. Acad. Sci. USA, 86, 1593 (1989)] can be used.

The methods for amplifying a foreign gene using a vector are illustrated in detail below.

The copy number of a unit of a foreign gene can be efficiently increased without using, for example, a special agent. It is accomplished by constructing a vector in which the recombination hot spot region and the autonomously replicating region are arranged close to the foreign gene to be amplified as above, introducing the vector into a host cell, introducing a vector in which a DNA encoding a protein having an activity to block replication fork progression was inserted into the host cell, and then expressing the protein, or by inserting the recombination hot spot region, the autonomously replicating region, and the DNA encoding a protein having the activity to block replication fork progression into the same expression vector and expressing the vector introduced into a host cell. Not only the copy number of the unit of the foreign gene can be increased, but also the protein encoded by the foreign gene can be effectively produced by using a vector which can express the foreign gene and which comprises a promoter and a ribosome binding sequence upstream of the foreign gene, and optionally transcription termination sequence downstream of it. By using this method, it is possible to efficiently screen a transformant with high productivity of the protein encoded by the foreign gene. As a promoter, a ribosome binding sequence and such, one can use those which originally belong to the foreign gene, or as well one can use recombinant ones that enhances the efficiency of expression. Such a promoter and a ribosome binding sequence are discussed below.

A method to construct an amplification vector for a foreign gene and to produce a protein encoded by the foreign gene by increasing the copy number of the unit of that foreign gene and a method to produce a protein encoded by that foreign gene utilizing an expression vector are illustrated below. An expression vector for producing a protein having the activity to block replication fork progression, and the production of that protein can be efficiently accomplished by the same method, and therefore, the method will be explained together below.

The protein encoded by the foreign gene can be produced by preparing a recombinant vector in which the recombination hot spot region and the autonomously replicating region are arranged close to the foreign gene comprising a promoter and ribosome binding sequence upstream and optionally a transcription termination sequence downstream as described above, introducing the vector into a host cell, obtaining a transformant which expresses the foreign gene, and culturing the transformant thereby enhancing the copy number of the unit of the foreign gene.

A protein having the activity to block replication fork progression can be produced by constructing a recombinant vector composed of a promoter, a ribosome binding sequence, the DNA encoding the protein, and a transcription termination sequence, introducing the vector into a host cell, obtaining transformants expressing the protein, and culturing the transformants.

As a host cell, any cell, for example, prokaryotic cell, such. as bacteria, or eukaryotic cell, such as yeast, animal cell, insect cell, and plant cell, can be used, as long as it can express the target gene.

Preferably, the expression vector comprises a gene for a marker, such as drug resistance and auxotrophy, for confirmation of the introduction of the gene.

In the case of using prokaryotic cells, for example E. coli, as the host cell, expression vectors, for example, pSE280 (Invitrogen), pGEMEX-1 (Promega), pQE-8 (Qiagen), pKYP10 (Unexamined Published Japanese Patent Application (JP-A) No. Sho 58-110600), pKYP200 [Agric. Biol. Chem., 48, 669 (1984)], pLSA1 [Agric. Biol. Chem., 53, 277 (1989)], pGELl [Proc. Natl. Acad. Sci., USA, 82, 4306 (1985)], pBluescript II SK (-) (STRATAGENE), pTrs30 (FERM BP-5407), pTrs32 (FERM BP-5408), pGHA2 (FERM BP-400), pGKA2 (FERM B-6798), pTerm2 (JP-A Hei 3-22979, USP4686191, USP4939094, USP5160735), pKK233-3 (Amersham Pharmacia Biotech), pGEX (Pharmacia), pET System (Novagen), pSupex, pTrxFus (Invitrogen), and pMAL-c2 (New England Biolabs), can be used.

Any promoter capable of expressing genes in a host cell can be used. In the case of using E. coli as a host, for example, a promoter derived from E. coli, phage, and so on, such as trp promoter (Ptrp), lac promoter (Plac), P_{L} promoter, T7 promoter, P_{R} promoter, can be used. An artificially designed and modified promoter and such, for example, a promoter in which two Ptrps are linked in tandem (Ptrp x 2), tac promoter, T7lac promoter, let I promoter, can be used. In the case of using Bacillus subtilis as a host, examples are promoters of SPO1 and SPO2, which are phage of Bacillus subtilis, and penP promoter.

As a ribosome binding sequence, a plasmid in which the distance between Shine-Dalgarno sequence and an initiation codon is appropriately adjusted (for example, 6 to 18 bases) is preferably used.

As a host cell, a microorganism belonging to, for example, the genus Escherichia, the genus Serratia, the genus Bacillus, the genus Brevibacterium, the genus Corynebacterium, the genus Microbacterium, and the genus Pseudomohas, such as Escherichia coli XL-1Blue, Escherichia coli XL2-Blue, Escherichia coli DH1, Escherichia coli MC1000, Escherichia coli KY3276, Escherichia coli W1485, Escherichia coli JM109, Escherichia coli HB101, Escherichia coli No. 49, Escherichia coli W3110, Escherichia coli NY49, Serratia ficaria, Serratia fonticola, Serratia liquefaciens, Serratia marcescens, Balillus subtilis, Bacillus amyloliquefaciens, Brevibacterium ammoniagenes, Brevibacterium immariophilum ATCC14068, Brevibacterium saccharolyticum ATCC14066, Corynebacterium glutamicum ATCC13032, Corynebacterium glutamicum ATCC14067, Corynebacterium glutamicum ATCC13869, Corynebacterium acetoacidophilum ATCC13870, Microbacterium ammoniaphilum ATCC15354, and Pseudomonas sp. D-0110, can be used.

As a method for introducing a recombinant vector, any method which introduces the DNA into the host cells described above can be used. Examples are, method using calcium ions [Proc. Natl. Acad. Sci. USA, 69, 2110 (1972)], protoplast method (JP-A Sho 63-24839), and electroporation method [Gene, 17, 107 (1982); Molecular & General Genetics, 168, 111 (1979)].

In the case of using a yeast strain as a host cell, an expression vector, for example YEp13 (ATCC37115), YEp24 (ATCC37051), YCpSO (ATCC37419), pHS19, and pHS15, can be used. These vectors can be modified for use by inserting a nucleotide sequence comprising the RFB region downstream of the cloning site.

Any promoter capable of expressing genes in yeast strains can be used. Examples are PH05 promoter, PGK promoter, GAP promoter, ADH promoter, gal 1 promoter, gal 10 promoter, heat shock polypeptide promoter, MFα1 promoter, and CUP 1 promoter.

As a host cell, a yeast strain belonging to, for example, the genus Saccharomyces, the genus Schizosaccharomyces, the genus Kluyveromyces, the genus Trichosporon, the genus Schwanniomyces, and the genus Pichia, and specifically, Saccharomyces serevisiae, Schizosaccharomyces pombe, Kluyveromyces lactis, Trichosporon pullulans, Schwanniomyces alluvius, and Pichia pastoris, can be used.

As a method for introducing a recombinant vector, any method for introducing a DNA into yeast, for example, electroporation method [Methods in Enzymology, 194, 182 (1990)], spheroplast method [Proc. Natl. Acad. Sci. USA, 81, 4889, (1984)], and lithium acetate method [Journal of Bacteriology, 153, 163 (1983)], can be used.

In case of using animal cells as the host, examples of expression vectors are pcDNAI/Amp (Invitrogen), pcDNAI, pAMoERC3Sc, pCDM8 [Nature, 329, 840 (1987)], pAGE107 [JP-A Hei 3-22979; Cytotechnology, 3, 133 (1990)], pREP4 (Invitrogen), pAGE103 [Journal of Biochemistry, 101, 1307 (1987)], pAMo, pAMoA, and pAS3-3 (JP-A Hei 2-227075).

Any promoter capable of expressing genes in animal cells, for example, sequence derived from viruses, such as a promoter and an enhancer for an immediate early (IE) gene of cytomegarovirus (CMV), early promoter of SV40, and a long terminal repeat on retroviruses, for example, Rous sarcoma virus (RSV), human immunodeficiency virus (HIV), and Molony mouse leukemia virus (MMLV); and promoter of genes derived from animal cells, such as metallothionein β-actin, elongation factor-1, and heat shock protein, can be used.

As animal cells, for example, mouse myeloma cells, rat myeloma cells, mouse hybridoma cells, Namalwa cells or Namalwa KJM-1 cells derived from human, human fetus kidney cells, human leukemia cells, African green monkey kidney cells, CHO cells derived from Chinese hamster, and HBT5637 (JP-A Sho 63-299) can be used.

As a mouse myeloma cell, for example, SP2/0 and NS0, as a rat myeloma cell, for example, YB2/0, as a human fetus kidney cell, for example, HEK293 (ATCC: CRL-1573) , as a human leukemia cell, for example, BALL-1, and as an African green monkey kidney cell, for example, COS-1 and COS-7, can be used.

As a method for introducing a recombinant vector, any method for introducing a DNA into an animal cell, for example, electroporation method [Cytotechnology, 3, 133 (1990)], calcium phosphate method (JP-A Hei 2-227075), lipofection method [Proc. Natl. Acad. Sci. USA, 84, 7413 (1987)], and a method described in Virology, 52, 456 (1973) can be used.

A vector for introduction of a foreign gene and an expression vector for production of a protein having the activity to block replication fork progression are introduced into a host cell in the above manner.

In case of inserting a DNA encoding a protein having the activity to block replication fork progression into the vector for introducing a foreign gene under the above-described conditions, only the vector for introducing a foreign gene is introduced into the host cell.

A transformant obtained as above can be cultured by the following method.

When the transformant is a prokaryote, such as E. coli, or an eukaryote, such as yeast, the medium for culturing this organism can be either a natural or a synthetic medium, as long as it comprises a carbon source, a nitrogen source, inorganic salts, and so on which can be assimilated by the organism and as long as it makes it possible to efficiently culture transformants.

As the carbon source, any source which can be utilized in each transformant, for example, glucose, fructose, sucrose, and molasses containing them, carbohydrate such as starch or starch hydrolyzate, organic acid, such as acetic acid and propionic acid, and alcohol such as ethanol and propanol, can be used.

As the nitrogen source, ammonium salt of various inorganic acids or organic acids, such as ammonia, ammonium chloride, ammonium sulfate, ammonium acetate, and ammnonium phosphate, other compounds containing nitrogen, peptone, meat extract, yeast extract, corn steep liquor, casein hydrolyzate, soybean cake and soybean cake hydrolyzate, cells of various fermentating microorganism, and their digests, and so on can be used.

As the inorganic salt, for example, potassium dihydrogen phosphate, dipotassium phosphate, magnesium phosphate, magnesium sulfate, sodium chloride, ferrous sulfate, manganese sulfate, copper sulfate, and calcium carbonate, can be used.

Cultivation is performed under aerobic conditions, such as shaking culture and deep ventilation stirring culture. Cultivation temperature is preferably 15 to 40°C, and cultivation time is ordinarily from 16 hours to 7 days. pH during cultivation should be maintained at 3.0 to 9.0. pH of a medium is adjusted, for example, with an inorganic or an organic acid, an alkaline solution, urea, calcium carbonate, or ammonia.

Antibiotics, such as ampicillin and tetracycline, may be added to the medium during culture if necessary.

To culture a microorganism transformed by an expression vector utilizing an inducible promoter as the promoter, an inducer may be added to the medium if necessary. For example, to culture a microorganism transformed by an expression vector with lac promoter, isopropyl-β-D-thiogalactopyranoside (IPTG) for example can be added to the medium. To culture a microorganism transformed by an expression vector with trp promoter, indole acrylic acid (IAA) for example can be added to the medium.

When the transformant is an animal cell, such medium generally used as RPMI1640 medium [The Journal of the American Medical Association, 199, 519 (1967)], Eagle's MEM medium [Science, 122, 501 (1952)], DMEM medium [Virology, 8, 396 (1959)], 199 medium [Proceeding of the Society for the Biological Medicine, 73, 1 (1950)], or these media supplemented with fetal calf serum for example, can be used for culturing the cell.

Cultivation is ordinarily done, for example, at pH 6 to 8 at 30 to 40°C in the presence of 5% CO₂ for 1 to 7 days.

Antibiotics, such as kanamycin and penicillin, may be added to the medium during cultivation if necessary.

As a medium for culturing a transformant obtained from an insect cell as a host cell, such medium generally used as TNM-FH medium (Pharmingen), Sf-900 II SFM medium (Life Technologies), ExCel1400, ExCel1405 [both manufactured by JRH Biosciences], and Grace's Insect Medium [Grace, T. C. C., Nature, 195, 788 (1962)] can be used.

Cells are cultured preferably at pH of 6 to 7, at a temperature of 25 to 30°C and ordinarily for 1 to 5 days.

Antibiotics, such as gentamycin, maybe added to the medium during cultivation if necessary.

### Brief Description of the Drawings

Figure 1 shows a structure of a repeated unit of the rRNA gene in yeast (S. cerevisiae).

Bold arrows indicate locations and directions of each rRNA gene 35S and 5S. White parts within the arrows show a transcribed spacer region digested during processing of the 35S rRNA.
NTS1, NTS2, two non-transcribed spacer regions;
ARS, Autonomously replicating sequence;
I-element, I element of HOT1;
E-element, E element of HOT1;
Enhancer, an enhancer of 35S rRNA transcription;
RFB, RFB site;
White boxes, sites amplified by competitive PCR conducted in Example 1 (2).

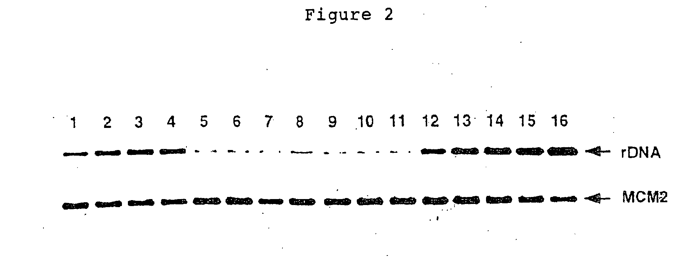

Figure 2 shows the results of Southern blot analysis of rRNA gene in the chromosomal DNA. Lane 1 shows the results for strain NOY408-1a, Lanes 2 to 4 for strain NOY408-1a/pNOY117 (44th, 80th, and 116th generations, respectively), Lane 5 for strain NOY408-1af, Lanes 6 to 8 for strain NOY408-1af/pNOY117 (44th, 80th, and 116th generations, respectively), Lanes 9 to 11 for strain NOY408-1af/pNOY117, YEplac195 (44th, 80th, and 116th generations, respectively), Lanes 12 to 14 for NOY408-1af/pNOY117, YEp-FOB1 (44th, 80th, arid 116th generations, respectively) and Lanes 15 and 16 for strain NOY408 and strain NOY408-1b.

rDNA with an arrow indicates band location of the rRNA gene, and MCM2 with an arrow indicates that of the MCM gene.

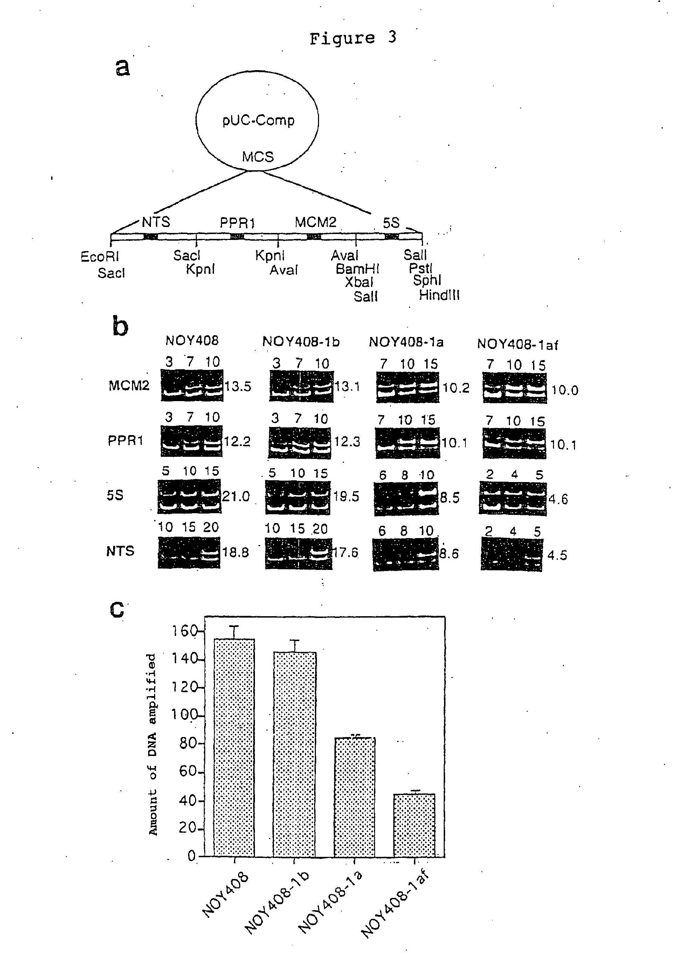

Figure 3 shows the result of competitive PCR for the rRNA gene using the chromosomal DNA as a template.
a: a figure showing the structure of pUC-Comp, a competitive DNA, used for competitive PCR.
b: the photograph of acrylamide gel electrophoresis of competitive PCR. Names of target genes to be amplified are indicated at left, and names of yeast strains used are indicated at the upper part. The lower band in each lane shows the amplified product of genomic DNA, and the upper one shows the amplified product of the competitive DNA. The number indicated above each lane is a quantitative ratio of added competitive DNA, and that indicated at right of each photograph is quantitative ratio of the calculated target DNA. Indicated numbers are multiplied by 10⁻⁴ times for MCM2 and PPR1, and 10⁻⁶ times for 5S and NTS.
c: a graph showing the copy numbers of the rRNA genes in each strain, obtained from the results of competitive PCR.

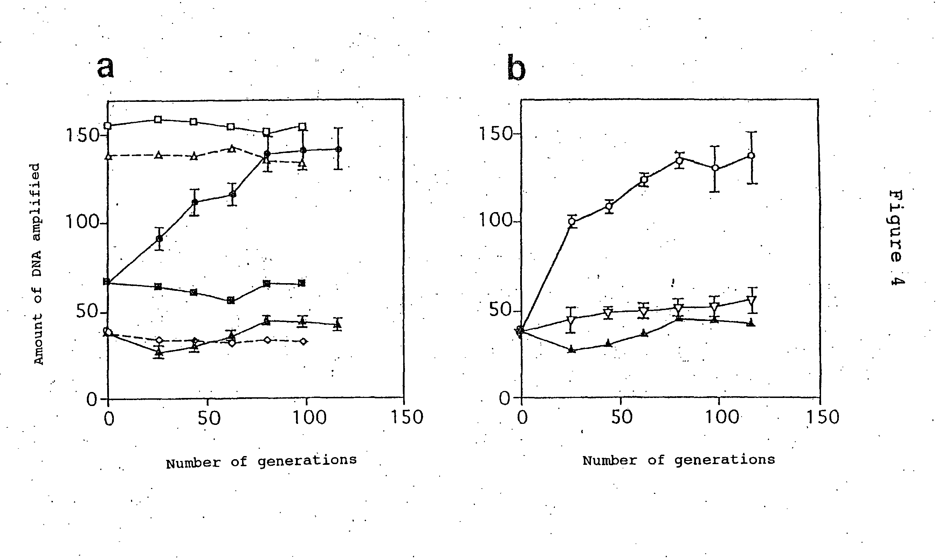

Figure 4 shows the time courses of the copy numbers of the rRNA genes per haploid genome. The horizontal axis indicates the number of generations. □ shows the results for strain NOY408, Δ for strain NOY408-1b, ■ for strain NOY408-1a, ◇ for strain NOY408-1af, ● for strain NOY408-1a/pNOY117, ▲ for strain NOY408-1af/pNOY117, ○ for strain NOY408-1af/pNOY117, YEp-FOB1, and ∇ for strain NOY408-1af/pNOY117, YEplac195.

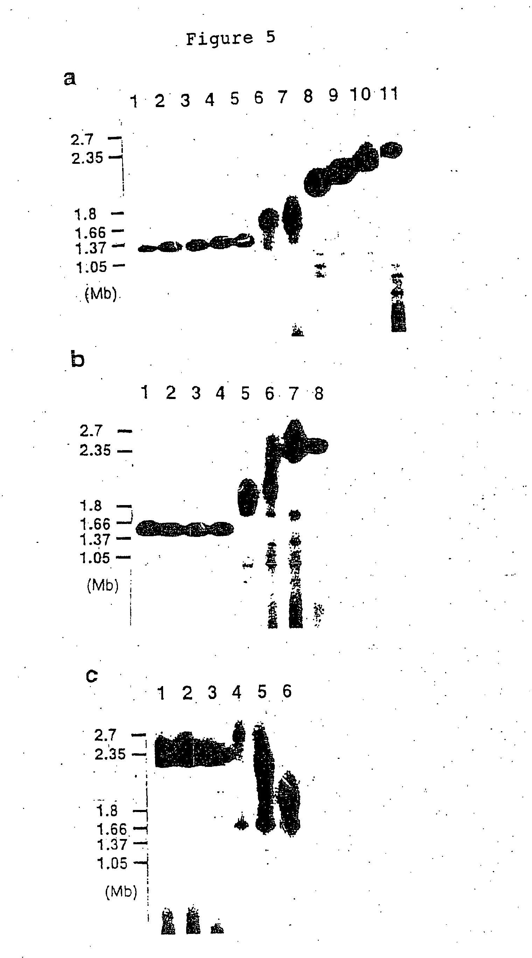

Figure 5 shows Southern blot analysis of pulse field gel electrophoresis of the chromosomal DNA. a and b show results of each generation after the introduction of the plasmids, and c shows the results in each generation after germination of asci.
a: Lanes 1 and 2 show the results for strain NOY408-1af (44th and 116th generations, respectively), lanes 3 to 5 for strain NOY408-1af/pNOY117 (44th, 80th, and 116th generations, respectively), lanes 6 and 7 for strain NOY408-1a (44th and 116th generations, respectively), lanes 8 to 10 for NOY408-1a/pNOY117 (44th, 80th, and 116th generations, respectively), and lane 11 for strain NOY408.
b: Lane 1 shows the results for strain NOY408-1af/pNOY117, lanes 2 to 4 for strain NOY408-1af/pNOY117, YEplacl95 (44th, 80th, and 116th generations, respectively), lanes 5 to 7 for strain NOY408-1af/pNOY117, YEp-FOB1 (44th, 80th, and 116th generations, respectively), and lane 8 for strain NOY408.
c: Lanes 1 to 3 show results for strain NOY408-2af (44th, 80th, and 116th generations, respectively) , and lanes 4 to 6 for strain NOY408-2a (44th, 80th, and 116th generations, respectively).

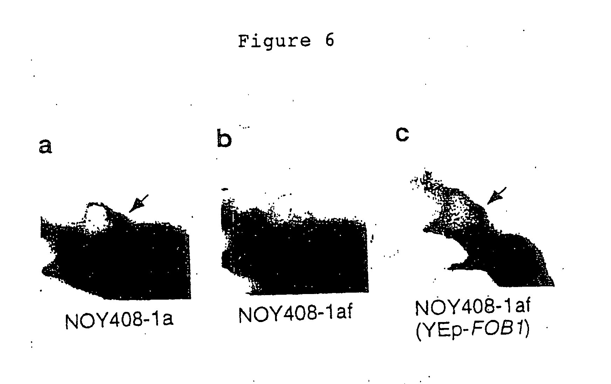

Figure 6 indicates detection of the fork blocking activity by Southern hybridization after 2D electrophoresis of chromosomal DNA. The arrow indicates a Y-form DNA.
a shows the results of strain NOY408-la, b for strain NOY408-1af, and c for strain NOY408-1af/YEp-FOB1.

### Best Mode for Carrying Out the Invention

The methods in Examples below were done following the description in Method in Yeast Genetics, Course Manual, Cold Spring Harbor Laboratory Press, NY (1994), or Molecular Cloning, Second Edition unless otherwisementioned. Strains which became auxotroph by plasmid introduction or gene disruption were cultured in a medium supplemented with 20 mg/L of required amino acids or adenine at 30°C.

### Example 1

### Decrease of the rRNA gene copy number in RNA polymerase I-defective mutant

The effect of deficiency of RNA polymerase I, which transcribed the 35S rRNA gene, on amplification of the rRNA genes was examined using an RNA polymerase I-defective mutant.

Yeast strain NOY408-1a was used as an RNA polymerase I-defective mutant.

The strain NOY408-1a is a mutant in which RPA135 gene encoding A135 subunit, the second largest subunit in RNA polymerase I, is disrupted and into which a plasmid pNOY102, which can express the 35S rRNA gene under the control of a promoter for GAL7 gene transcribed by RNA polymerase II, was introduced. Transcription of the 35S rRNA gene dependent on the RNA polymerase I does not occur due to the disruption of the RPA135 gene, which makes growth of the strain impossible. However, introduction of the pNOY102 enables expression of the 35S rRNA gene, and thus growth of the strain. The GAL7 promoter is induced by galactose, and therefore, the strain NOY408-1a can grow in a medium containing galactose, but in a medium containing glucose instead of galactose, the growth is stopped.

The strain NOY408-1a is a haploid obtained from a diploidparental strain NOY408 by the dissection of ascospores. The diploid parental strain NOY408 [Proc. Nail. Acad. Sci. USA, 88, 3962 (1991) ; genotype, MATa/MATα ade2-1/ade2-1 ura3-1/ura3-1 his3-11/his3-11 trp1-1/trp1-1 leu2-3, 112/leu2-3, 112 can1-100/can1-100 rpa135::LEU2/RPA135 pNOY102] has a wild-type RPA135 gene, an RPA135 gene disrupted by the insertion of the LEU2 gene (rpa135::LEU2), and pNOY102. As control strains comprising the wild-type RPA135 gene, the strain NOY408 and the strain NOY408-1b obtained simultaneously by the dissection of ascospores [Proc. Natl. Acad. Sci. USA, 88, 3962 (1991); genotype, MATa ade2-1 ura3-1 his3-11 trp2-3, 112 can1-100 pNOY102] were used for comparison.

### (1) Southern blot analysis

The strain NOY408-1a was cultured in the YEp-galactose medium containing galactose [Method in Yeast Genetics, Course Manual, Cold Spring Harbor Laboratory Press, NY (1994)] at 30°C and chromosomal DNA was extracted by a standard method. The chromosomal DNA was digested with restriction enzyme BglII and subjected to agarose gel electrophoresis. The agarose gel was blotted on a membrane for Southern blot analysis of the rRNA gene. A DNA fragment (about 1.2 kb) , obtained by extracting chromosomal DNA from the strain NOY408-1a and digesting the DNA with restriction enzymes HindIII and SphI, was labeled with ³²P and used as a probe specific for the rRNA gene. The 4.6 kb band derived from the rRNA gene was detected by hybridizing the membrane described above with the probe. The bands were detected and quantified by Bio Imaging Analyzer BAS2000 (Fuji Photo Film). The MCM2 gene [the gene involved in replication initiation: Genes & Development, 5, 944 (1991)], which existed as one copy per haploid genome, was analyzed by Southern blot analysis in the same manner for further quantifying the bands. By using the quantity of bands of the MCM2 gene as an internal standard, the quantity of bands of the rRNA gene was corrected, and the relative copy numbers were compared. Figure 2 shows the result. Under conditions in which the quantity of bands of the MCM2 gene was approximately the same, the copy number of the rRNA gene of strain NOY408-1a, a RPA135 gene defective mutant, decreased to about one half of the copy numbers of the strain NOY408 and NOY408-1b, comprising wild-type RPA135 gene.

### (2).Analysis by competitive PCR

Competitive PCR was conducted to evaluate more precise absolute value of copy numbers. The copy numbers of the rRNA gene were measured for each 5S rRNA gene region and NTS2 region in the rRNA gene. As internal standards of a single copy, the PPR1 gene, which is a transcriptional factor stimulating transcription of enzyme genes URA1 and URA3 in the pyrimidine biosynthesis pathway [Mol. Gen. Gen., 184, 394 (1981); J. Mol. Biol., 180, 239 (1984)], and the MCM2 gene were used. Four specific primers each (1, 2, 3, and 4) were designed and synthesized for preparing competitive DNA of these four target DNAs to be amplified. (Nucleotide sequences are shown in SEQ ID NOs: 1 to 16. 1 to 4, 5S rRNA gene-specificprimers 5S-1 to 5S-4, respectively; 5 to 8, NTS2-specific primers NTS-1 to NTS-4, respectively; 9 to 12, PPR1 gene-specific primers PPR-1 to PPR-4, respectively; 13 to 16, MCM2 gene-specific primers MCM-1 to MCM-4, respectively) Primers 1 and 4 are external primers, in which a sequence of restriction enzyme site (the 5S rRNA gene, SalI; NTS2, SacI; PPR1 gene, KpnI; the MCM2 gene, AvaI) is added to the 5' end. Primers 2 and 3 are internal primers, in which a sequence of 20 nucleotides derived from λ phage is added to the 5' end. A competitive DNA was prepared according to the reference [Gene, 122, 313 (1992)] as follows. PCR was conducted with the chromosomal DNA of the yeast strain NOY408 as the template separately using primers 1 and 2 and primers 3 and 4. Two types of fragments were amplified. Accordingly one strand of one type of amplified fragments was annealed with one strand of the other type of amplified fragments using the sequence derived from λ phage located at each 3' end, and this was used as both the primer and the template to conduct PCR to amplify the nucleotide sequence in which 20 bp derived from λ phage is inserted at the center. Moreover, these four types of amplified fragments were digested at the restriction enzyme site derived from each external primer sequence, arranged at the multicloning site of the vector pUC18 plasmid, and cloned to prepare plasmid pUC-Comp (Figure 3a) for using as a competitive DNA.

PCR was conducted using this competitive DNA and primers A and B in which a sequence of restriction enzyme site was removed from primers 1 and 4, respectively. (The nucleotide sequences are shown in SEQ ID NOs: 17 to 24. SEQ ID NOs: 17 and 18, 5S rRNA gene-specific primers; SEQ ID NOs: 19 and 20, NTS2-specific primers; SEQ ID NOs: 21 and 22, PPR1 gene-specific primers; SEQ ID NOs: 23 and 24, MCM2 gene-specific primers) A fragment only 20 bp longer than the PCR product obtained using the genomic DNA due to the insertion in the center was amplified. Concentration of the pUC-Comp was measured by absorbance at 260 nm, and the pUC-Comp was used for competitive PCR. The competitive PCR was conducted under the following conditions.

Whole genomic DNA was extracted from each yeast in which the copy number was to be measured and digested with EcoRI to use as a template. PCR with 30 cycles (reaction of one cycle was composed of denaturation at 94°C for 30 sec, annealing at 60°C for 30 sec, and elongation at 72°C for 30 sec) was conducted with various concentrations of competitive DNA pUC-Comp, and primer A and B, using AmpliTaq Gold Taq polymerase (Perkin-Elmer) heated for 8 min at 95°C. The amplified DNA fragment was separated by 10% polyacrylamide gel electrophoresis, and the gel was stained with 0.5 mg/ml ethidium bromide. The DNA was then quantified with a densitometer. The number of the target gene molecules was determined by the ratio of quantity of the competitive DNA to that of the target DNA (Figure 3b).

The copy number of the rRNA gene per haploid genome was calculated by calculating the average of the number of molecules in 5S and NTS2 as the number of the rRNA gene molecule, and dividing this average by the average number of the MCM2 and the PPR1 molecules as the number of gene molecules in one copy per haploid genome. The copy number of the rRNA gene for the RPA135 gene mutant NOY408-1a was about 80 copies per haploid cell, and this was about one half of the copy number 150 for the strain NOY408 and the strain NOY408-1b comprising the normal RPA135 gene (Figure 3c).

### Example 2

Recovery of decrease in the rRNA gene copy number by introduction of the RPA135 gene

### (1) Southern blot analysis

To confirm that the decrease in the rRNA gene copy number in the strain NOY408-1a observed in Example 1 was due to defective mutation of the RPA135 gene, the rRNA gene copy number in strain NOY408-1a with RPA135 gene expression was examined. The strain NOY408-1a was transformed by lithium acetate method using the wild-type RPA135 gene expression multi-copy plasmid pNOY117. Four clones per one strain were selected and single-cloned to examine the relationship between division frequency and the copy number after the introduction of the gene. Four colonies with a diameter of approximately 1 mm per one clone were collected again, mixed, and single-cloned. This procedure was repeated. It was estimated that in a single colony, there exist cells multiplied 10⁵ times by 18 division cycles from a purified cell. Finally four colonies per one clone were selected and cultured in 2 ml of a liquid medium. The number of generation was calculated by measuring turbidity at a wavelength of 600 nm. Chromosomal DNA was extracted from cells at several generations up to 116th generation after transformation, and the copy number was measured by Southern blot analysis of rRNA gene in the same manner as Example 1 (1).

As shown in lanes 2 to 4 in Figure 2, in strain NOY408-1a into which the plasmid pNOY117 was introduced, the quantity of bands for the rRNA gene, namely the copy number, gradually increased as the generation altered to 44th, 80th, and 116th. During this period, the copy number of the MCM2 gene, an internal standard, did not change. The copy number of the rRNA gene in the strain NOY408-1b comprising the wild-type RPA135 gene was estimated to be 156 copies in Example 1 (2). The copy number of the rRNA gene in each clone was calculated from the corrected quantity of bands for the rRNA gene according to this estimation, and the average number among four clones was used as the copy number of the rRNA gene. As a result, it was observed that the copy number of the rRNA gene in strain NOY408-1a into which the plasmid pNOY117 was introduced (● in Figure 4), gradually increased as the generation altered, and recovered to the level of the control strain NOY408 (□ in Figure 4) and NOY408-1b (Δ in Figure 4) comprising the wild-type RPA135 gene at around the 80th generation. The strain NOY408 and the strain NOY408-1b, and the strain NOY408-1a into which the pNOY117 was not introduced (■ in Figure 4) did not show any change of copy numbers even after cultivation for 100 generations or more.

### (3) Analysis by pulse field gel electrophoresis

The yeast rRNA gene is known to be located on chromosome XII. The copy number of the rRNA gene was analyzed by measuring the size of chromosome XII using pulse field electrophoresis. Extraction of yeast chromosomal DNA and pulse field electrophoresis were conducted based on the method by Smith et al. (In Genome Analysis, IRL Press, p41-112 (1988)). Pulse field gel electrophoresis was conducted for the same yeast cells as those used in Southern blot analysis of (1), using an electrophoretic device CHEF-DRII (BioRad), under the conditions of pulse time of 300 to 900 seconds, electrophoresis duration of 68 hours, 100 CV, at 14°C in 0.5 X TBE buffer. Accordingly Southern blot analysis using the rRNA gene fragment as a probe was performed in the same manner as (1) to detect chromosome XII. It was known that the length of chromosome XII except for the part of the rRNA gene is 1.0 Mbp, and the length of a repeated unit of the rRNA gene (1 copy) is 9.1 kb. The copy number of the rRNA gene can be calculated from the length of chromosome XII.

The length of chromosome XII of NOY408-1a, which is the RPA135 gene-defective strain, is 1.1 Mbp to 1.9 Mbp as shown in lanes 6 and 7 in Figure 5a, which is shorter compared to about 2.4 Mbp of chromosome XII length in the parental strain NOY408 comprising the normal RPA gene (lane 11 in Figure 5a). The copy number was calculated as 11 to 90 copies. It was confirmed that the length of chromosome XII elongated with the progression of generations after the introduction of the RPA135 gene expression plasmid pNOY117 into the strain NOY408-1a (lanes 8 to 10 in Figure 5a). The length of chromosome XII corresponding to copy number of rRNA gene therein were 2.0 Mbp with 109 copies in the 44th generation (lane 8), 2.2 Mbp with 132 copies in the 80th generation (lane 9), and 2.4 Mbp with 154 copies in the 116 generation (lane 10).

### Example 3

### Gene amplification of the rRNA gene in the replication fork blocking-defective strain

It has been reported that the activity of replication fork blocking does not depend on the transcription of the 35S rRNA [Mol. Gen. Genet., 233, 355 (1992); Cell, 71, 267 (1992)]. Involvement of the FOB1 gene in gene amplification of the rRNA gene was examined.

### (1) The activity of replication fork blocking dependent on the FOB1 gene of the RPA135 gene-defective strain

The activity of replication fork blocking at the RFB site within the rRNA gene cluster of strain NOY408-la was examined based on the method in reference [Cell, 51, 463 (1987)]. Chromosomal DNA was extracted from strain NOY408-1a by a standard method, digested with restriction enzymes BglII and SphI, and the DNA fragments were subjected to agarose gel 2D electrophoresis. The agarose gel was blotted on a membrane for Southern blot analysis of the rRNA gene in the same manner as in Example 1 (1). As a result, a Y-form DNA spot specifically formed at the termination of replication fork at the RFB site [Cell, 71, 267 (1992)] was observed, confirming that the strain NOY408-1a comprises the activity of replication fork blocking (Figure 6a).

A strain in which the FOB1 gene in strain NOY408-1a is disrupted (described as NOY408-1af) was prepared as follows. The HIS3 gene was amplified by PCR using plasmid pJJ214 with the HIS3 gene [Yeast, 6, 363 (1990)] as a template and the HIS3 gene specific primers comprising the nucleotide sequence of NruI or ClaI site at each 5' end. Plasmid pUC-fob1::HIS3, in which the FOB1 gene was disrupted, was prepared by inserting the HIS3 gene fragment obtained by digesting the ends of the amplified products with NruI and ClaI between NruI/ClaI within the FOB1 gene of the plasmid pUC-FOB1 [Genes to Cells, 1, 465 (1996)]. This plasmid pUC-fob1::HIS3 was linearized by digestion with EcoRI, and introduced into the strain NOY408-1a by lithium acetate method. The strain NOY408-1a, which comprised his3-11 mutation and which was histidine-auxotrophic, was transformed into histidine-prototrophic cells by replacement of the FOB1 gene with fob1::HIS3 by homologous recombination. By screening such cells, the strain NOY408-1af, in which the FOB1 gene was disrupted, was obtained. The activity of replication fork blocking in the strain NOY408-1af was examined in the same manner described above.. It was confirmed that the Y-form DNA spots completely disappeared and that it was defective in the fork blocking activity (Figure 6b).

The FOB1 gene expression plasmid YEp-FOB1 was prepared by inserting the FOB1 gene fragment amplified by PCR in the same manner described in reference [Genes to Cells, 1, 465 (1996)] into the BamHI site in the yeast expression vector YEplacl95 [Gene, 74, 527 (1988)]. The replication fork-blocking activity in the strain obtained by transforming the strain NOY408-1af with the plasmid was examined in the same manner described above. The Y-form DNA spots were observed again, confirming the recovery of the replication fork blocking activity (Figure 6c).

These results confirmed that the activity of replication fork blocking at the RFB site in the rRNA gene in RNA polymerase I-defective strain was also dependent on the FOB1 gene.

### (2) Southern blot analysis

The copy number of the rRNA gene in the strain NOY408-1af, in which both the FOB1 gene and RPA135 gene were mutated, was analyzed and quantified by Southern hybridization at each generation in the same manner as in Example 2. The copy number of the rRNA gene decreased to about the half of that of RPA135 gene-mutant strain NOY408-1a (lane 5 in Figure 2 and ◇ in Figure 4). The copy number did not change even after 100 generations. In the case of introducing the RPA135 expression plasmid pNOY117 into strain NOY408-1af, unlike strain NOY408-1a, the copy number of the rRNA gene did not recover even after 100 generations (lanes 6 to 8 in Figure 2 and ◇ in Figure 4). However, when the FOB expression plasmid YEp-FOB1 was further introduced into the strain NOY408-1af transformed with pNOY117, increase in the copy number of the rRNA gene was observed (lanes 12 to 14 in Figure 2). Quantification of the copy numbers revealed that the copy number gradually increased at a rate of about 1 copy per generation, and the increase terminated about at the 80th generation. No increase was observed thereafter (○ in Figure 4). Increase in the copy number was not observed when the vector YEplacl95 without the FOB1 gene was introduced (lanes 9 to 11 in Figure 2 and ∇ in Figure 4). These results revealed that amplification of the rRNA gene required not only RNA polymerase I, but also blocking elongation of replication fork at the RFB site in the repetitive structure of the rRNA gene by the FOB1 gene product.

### (3) Analysis by pulse field gel electrophoresis

The length of chromosome XII in strain NOY408-1af in which both RPA135 gene and FOB1 gene were defective was detected by Southern blot analysis after pulse field gel electrophoresis in the same manner as in Example 2. The length was confirmed to be 1.34 Mbp, shorter than strain RPA135 gene defective NOY408-1a, and it contained about 40 copies of the repeated rRNA genes (lanes 1 and 2 in Figure 5a).

The length of chromosome XII did not change even if the RPA135 gene expression plasmid pNOY117 was introduced into the strain NOY408-1af and cultured until the 116th generation (lanes 3 to 5 in Figure 5a and lane 1 in Figure 5b). However, elongation of the length of chromosome XII was observed as proliferation progressed through the 44th, 80th, and 116th generations when the FOB1 gene expression plasmid YEp-FOB1 was further introduced (lanes 5 to 7 in Figure 5b). Although the elongation was more fluctuated compared to the case in which the pNOY117 was introduced into strain NOY408-1a, at the 116th generation it showed almost the same length as the parental strain NOY408 (lane 8 in Figure 5b). When the vector YEplac195 was introduced instead of the YEp-FOB1, the length of chromosome XII did not change even after proliferation till the 116th generation (lanes 2 to 4 in Figure 5b).

### Example 4

### Involvement of the FOB1 gene in decreased copy number of the rRNA gene in RNA polymerase I-defective mutant

Two clones of haploid segregant [rpa135, fob1] in which both RPA gene and FOB1 gene were defective were newly isolated by dissection of ascospores of the FOB1 gene homozygously defective diploid strain NOY408-f [RPA135/rpa135, fob1/fob1] prepared by disrupting the FOB1 gene in the strain NOY408. These are designated NOY408-2af. The genotype of this strain is the same as that of NOY408-1af. A clone of the RPA135 gene-defective haploid strain [rpa135, FOB1] was newly isolated by dissection of ascospores from the NOY408 strain [RPA135/rpa135, FOB1/FOB1] in the same manner and was designated NOY408-2a. The genotype of this strain is the same as NOY408-1a. Cells of each strain were proliferated from the first asci, and chromosomal DNA was extracted from the cells at several generations calculated from the first asci. The length of chromosome XII was detected in the same manner as in Example 1. The copy number of strain NOY408-2a comprising the wild-type EOB1 gene was the same as that of the wild-type strain at the early stage (the 44th generation), but decreased as the proliferation progressed to the 80th and 116th generations (lanes 4 to 6 in Figure 5c). In contrast, the length of the chromosome in strain NOY408-2af in which the FOB1 gene was also defective did not change (lanes 1 to 3 in Figure 5c). These results indicate that the FOB1 gene is necessary for not only gene amplification, but also the decrease in the copy number of the rRNA gene.

### Industrial Applicability

A method for amplifying a foreign gene by arranging a recombination hot spot region and an autonomously replicating sequence close to a foreign gene and expressing a protein having an activity to block replication fork progression is disclosed. The application of this method to a recombinant gene amplification system as a novel system for amplifying a recombinant gene free of agents for gene amplification, enables efficient screening of cells with high productivity of recombinant proteins.

## Claims

1. A method for amplifying a foreign gene, the method comprising arranging a recombination hot spot region and an autonomously replicating sequence close to a foreign gene and expressing a protein having an activity to block replication fork progression.

2. The method of claim 1, wherein the recombination hot spot region and the autonomously replicating sequence is arranged upstream and/or downstream of the foreign gene.

3. The method of claim 1, wherein the recombination hot spot region is derived from HOT1.

4. The method of claim 1 or 2, wherein the recombination hot spot region comprises a replication fork blocking (RFB) region.

5. The method of claim 1, wherein the recombination hot spot region comprises a DNA replication terminus (Ter).

6. The method of claim 1, wherein the protein having an activity to block replication fork progression is a protein comprising the amino acid sequence of SEQ ID NO: 25 or 26, or a protein comprising the amino acid sequence in which one or several amino acids are deleted, replaced, or added and having the activity to block replication fork progression.

7. A protein comprising the amino acid sequence of SEQ ID NO: 25 or 26 in which one or several amino acids are deleted, replaced, or added and having an activity to block replication fork progression.

8. A DNA encoding the protein of claim 7.

9. A DNA hybridizing to the DNA of claim 8 under stringent conditions and encoding a protein having an activity to block replication fork progression.

10. A DNA hybridizing to the DNA of SEQ ID NO: 27 or 28 under stringent conditions and encoding a protein having an activity to block replication fork progression.

11. A recombinant vector obtained by inserting the DNA of any one of claims 8 to 10 into a vector and capable of expressing a protein encoded by the DNA.

12. An amplification vector for a foreign gene, the vector obtained by arranging, in an expression vector for a foreign gene, a recombination hot spot region and an autonomously replicating sequence close to a foreign gene.

13. The amplification vector of claim 12, wherein the recombination hot spot region and the autonomously replicating sequence is arranged upstream and/or downstream of the foreign gene.

14. The amplification vector of claim 12, wherein the recombination hot spot region is derived from HOT1.

15. The amplification vector of claim 12 or 13, wherein the recombination hot spot region comprises a replication fork blocking (RFB) region.

16. The amplification vector of claim 12, wherein the recombination hot spot region comprises a DNA replication terminus (Ter).

17. The amplification vector of any one of claims 12 to 16, wherein the vector comprises the DNA of any one of claims 8 to 10.

18. A transformant obtained by introducing vectors of any one or more of claims 12 to 17 into a host cell.

19. Amethod for amplifying a foreign gene, the method comprising using the transformant of claim 18.

20. A method for producing a protein encoded by a foreign gene, the method comprising using the transformant of claim 18.


SEQUENCE LISTING

<110> HORIUCHI, Takashi
KYOWA HAKKO KOGYO CO., LTD

<120> Method for amplifying foreign genes

<130> 11164

<160> 28

<170> PatentIn Ver. 2.0

<210> 1
<211> 28
<212> DNA
<213> Artificial Sequence

<220>
<223> Description of Artificial Sequence : Synthetic DNA

<400>1
gcgtcgacgt tgcggccata tctaccag 28

<210> 2

<211> 40
<212> DNA
<213> Artificial Sequence

<220>
<223> Description of Artificial Sequence : Synthetic DNA

<400>2
ccagcctcgc atatgaccaa taccagctta actacagttg 40

<210> 3
<211> 40
<212> DNA
<213> Artificial Sequence

<220>
<223> Description of Artificial Sequence : Synthetic DNA

<400>3
ttcctgatat gcgaggctgg agagcctgac cgagtagtgt 40

<210> 4
<211> 28
<212> DNA
<213> Artificial Sequence

<220>
<223> Description of Artificial Sequence : Synthetic DNA

<400>4
gcgtcgacag attgcagcac ctgagttt 28

<210> 5
<211> 28
<212> DNA
<213> Artificial Sequence

<220>
<223> Description of Artificial Sequence : Synthetic DNA

<400>5
gcgtcgacgt tgcggccata tctaccag 28

<210> 6
<211> 40
<212> DNA
<213> Artificial Sequence

<220>
<223> Description of Artificial Sequence : Synthetic DNA

<400>6

ccagcctcgc atatgaccaa taccagctta actacagttg 40

<210> 7
<211> 40
<212> DNA
<213> Artificial Sequence

<220>
<223> Description of Artificial Sequence : Synthetic DNA

<400>7
ccatcagata tcgttccgtc aatccatgcc ataacaggaa 40

<210> 8
<211> 28
<212> DNA
<213> Artificial Sequence

<220>
<223> Description of Artificial Sequence : Synthetic DNA

<400>8
gggagctctg aatagacata ggagtaag 28

<210> 9

<211> 28

<212> DNA

<213> Artificial Sequence

<220>

<223> Description of Artificial Sequence : Synthetic DNA

<400> 9

ggggtaccat aaggagatca gtgcgctg                                28

<210> 10

<211> 40

<212> DNA

<213> Artificial Sequence

<220>

<223> Description of Artificial Sequence : Synthetic DNA

<400>10

ccagcctcgc atatgaccaa taccagctta actacagttg                   40

<210> 11

<211> 40

<212> DNA

<213> Artificial Sequence

<220>
<223> Description of Artificial Sequence : Synthetic DNA

<400>11
tggctacgtc ctgatgcagg gcaactaatt ttcgtcaaga 40

<210> 12
<211> 28
<212> DNA
<213> Artificial Sequence

<220>
<223> Description of Artificial Sequence : Synthetic DNA

<400>12
ggggtacctg gattgttttc agcctctg 28

<210> 13
<211> 27
<212> DNA
<213> Artificial Sequence

<220>
<223> Description of Artificial Sequence : Synthetic DNA

<400> 13

ccccgggcgt agacgtgagg aagatga 27

<210> 14
<211> 40
<212> DNA
<213> Artificial Sequence

<220>
<223> Description of Artificial Sequence : Synthetic DNA

<400>14
tgatttgtca aacgcctgcc accaataggt gatgaaactg 40

<210> 15
<211> 40
<212> DNA
<213> Artificial Sequence

<220>
<223> Description of Artificial Sequence : Synthetic DNA

<400>15
ggcaggcgtt tgacaaatca tctggagata tgattaaccc 40

<210> 16
<211> 27

<212> DNA
<213> Artificial Sequence

<220>
<223> Description of Artificial Sequence : Synthetic DNA

<400>16
ccccgggcgt ccatcaaatc tacttcg                                27

<210> 17
<211> 20
<212> DNA
<213> Artificial Sequence

<220>
<223> Description of Artificial Sequence : Synthetic DNA

<400>17
gttgcggcca tatctaccag                                        20

<210> 18
<211> 20
<212> DNA
<213> Artificial Sequence

<220>

<223> Description of Artificial Sequence : Synthetic DNA

<400>18
agattgcagc acctgagttt                                    20

<210> 19
<211> 20
<212> DNA
<213> Artificial Sequence

<220>
<223> Description of Artificial Sequence : Synthetic DNA

<400>19
gttgcggcca tatctaccag                                    20

<210> 20
<211> 20
<212> DNA
<213> Artificial Sequence

<220>
<223> Description of Artificial Sequence : Synthetic DNA

<400>20
tgaatagaca taggagtaag 20

<210> 21
<211> 20
<212> DNA
<213> Artificial Sequence

<220>
<223> Description of Artificial Sequence : Synthetic DNA

<400> 21
ataaggagat cagtgcgctg 20

<210> 22
<211> 20
<212> DNA
<213> Artificial Sequence

<220>
<223> Description of Artificial Sequence : Synthetic DNA

<400>22
tggattgttt tcagcctctg 20

<210> 23
<211> 20
<212> DNA
<213> Artificial Sequence

<220>
<223> Description of Artificial Sequence : Synthetic DNA

<400> 23
cgtagacgtg aggaagatga                                    20

<210> 24
<211> 20
<212> DNA
<213> Artificial Sequence

<220>
<223> Description of Artificial Sequence : Synthetic DNA

<400>24
cgtccatcaa atctacttcg                                    20

<210> 25
<211> 566
<212> PRT

<213> Saccharomyces cerevisiae

<400> 25

Met Thr Lys Pro Arg Tyr Asn Asp Val Leu Phe Asp Asp Asp Asp Ser
1               5                   10                  15

Val Pro Ser Glu Ser Val Thr Arg Lys Ser Gln Arg Arg Lys Ala Thr
            20                  25                  30

Ser Pro Gly Glu Ser Arg Glu Ser Ser Lys Asp Arg Leu Leu Ile Leu
        35                  40                  45

Pro Ser Met Gly Glu Ser Tyr Thr Glu Tyr Val Asp Ser Tyr Leu Asn
    50                  55                  60

Leu Glu Leu Leu Glu Arg Gly Glu Arg Glu Thr Pro Ile Phe Leu Glu
65                  70                  75                  80

Ser Leu Thr Arg Gln Leu Thr Gln Lys Ile Tyr Glu Leu Ile Lys Thr
                85                  90                  95

Lys Ser Leu Thr Ala Asp Ala Leu Gln Gln Ile Ser Asp Lys Tyr Asp
            100                 105                 110

Gly Val Val Ala Glu Asn Lys Leu Leu Phe Leu Gln Arg Gln Tyr Tyr
        115                 120                 125

Val Asp Asp Glu Gly Asn Val Arg Asp Gly Arg Asn Asn Asp Lys Ile

    130                 135                 140

Tyr Cys Glu Pro Lys His Val Tyr Asp Met Val Met Ala Thr His Leu
145                 150                 155                 160

Met Asn Lys His Leu Arg Gly Lys Thr Leu His Ser Phe Leu Phe Ser
                165                 170                 175

His Phe Ala Asn Ile Ser His Ala Ile Ile Asp Trp Val Gln Gln Phe
            180                 185                 190

Cys Ser Lys Cys Asn Lys Lys Gly Lys Ile Lys Pro Leu Lys Glu Tyr
        195                 200                 205

Lys Arg Pro Asp Met Tyr Asp Lys Leu Leu Pro Met Glu Arg Ile His
    210                 215                 220

Ile Glu Val Phe Glu Pro Phe Asn Gly Glu Ala Ile Glu Gly Lys Tyr
225                 230                 235                 240

Ser Tyr Val Leu Leu Cys Arg Asp Tyr Arg Ser Ser Phe Met Trp Leu
                245                 250                 255

Leu Pro Leu Lys Ser Thr Lys Phe Lys His Leu Ile Pro Val Val Ser
            260                 265                 270

Ser Leu Phe Leu Thr Phe Ala Arg Val Pro Ile Phe Val Thr Ser Ser
        275                 280                 285

Thr Leu Asp Lys Asp Asp Leu Tyr Asp Ile Cys Glu Glu Ile Ala Ser
    290                 295                 300

Lys Tyr Gly Leu Arg Ile Gly Leu Gly Leu Lys Ser Ser Ala Arg Phe
305                 310                 315                 320

His Thr Gly Gly Ile Leu Cys Ile Gln Tyr Ala Leu Asn Ser Tyr Lys
                325                 330                 335

Lys Glu Cys Leu Ala Asp Trp Gly Lys Cys Leu Arg Tyr Gly Pro Tyr
            340                 345                 350

Arg Phe Asn Arg Arg Arg Asn Lys Arg Thr Lys Arg Lys Pro Val Gln
        355                 360                 365

Val Leu Leu Ser Glu Val Pro Gly His Asn Ala Lys Phe Glu Thr Lys
    370                 375                 380

Arg Glu Arg Val Ile Glu Asn Thr Tyr Ser Arg Asn Met Phe Lys Met
385                 390                 395                 400

Ala Gly Gly Lys Gly Leu Ile Tyr Leu Glu Asp Val Asn Thr Phe Ala
                405                 410                 415

Leu Ala Asn Glu Ala Asp Asn Ser Cys Asn Asn Asn Gly Ile Leu His
            420                 425                 430

Asn Asn Asn Ile Gly Asn Asp Asn Phe Glu Glu Glu Val Gln Lys Gln
        435                 440                 445
Phe Asp Leu Thr Glu Lys Asn Tyr Ile Asp Glu Tyr Asp Asp Leu Ala
    450                 455                 460
His Asp Ser Ser Glu Gly Glu Phe Glu Pro Asn Thr Leu Thr Pro Glu
465                 470                 475                 480
Glu Lys Pro Pro His Asn Val Asp Glu Asp Arg Ile Glu Ser Thr Gly
                485                 490                 495
Val Ala Ala Pro Met Gln Gly Thr Glu Glu Pro Glu Lys Gly Asp Gln
            500                 505                 510
Lys Glu Ser Asp Gly Ala Ser Gln Val Asp Gln Ser Val Glu Ile Thr
        515                 520                 525
Arg Pro Glu Thr Ser Tyr Tyr Gln Thr Leu Glu Ser Pro Ser Thr Lys
    530                 535                 540
Arg Gln Lys Leu Asp Gln Gln Gly Asn Gly Asp Gln Thr Arg Asp Phe
545                 550                 555                 560
Gly Thr Ser Met Glu Leu
                565
<210> 26
<211> 309

<212> PRT

<213> Escherichia coli

<400> 26

<210> 27

<211> 1698

<212> DNA

<213> Saccharomyces cerevisiae

<220>

<221> CDS

<222> (1)..(1698)

<400> 27

atg acg aaa ccg cgt tac aat gac gtg ttg ttt gat gat gat gac tcg 48

Met Thr Lys Pro Arg Tyr Asn Asp Val Leu Phe Asp Asp Asp Asp Ser

1               5                   10                  15

gta cca tca gaa tca gtt acg agg aaa tcg cag aga aga aag gca acg 96

Val Pro Ser Glu Ser Val Thr Arg Lys Ser Gln Arg Arg Lys Ala Thr

            20                  25                  30

tca cct ggg gaa tca aga gag tcc tca aaa gat cgt cta ctg ata ctt 144

Ser Pro Gly Glu Ser Arg Glu Ser Ser Lys Asp Arg Leu Leu Ile Leu

        35                  40                  45

ccc tct atg ggg gaa tca tat act gag tac gta gac tct tat ttg aac 192
Pro Ser Met Gly Glu Ser Tyr Thr Glu Tyr Val Asp Ser Tyr Leu Asn
    50                  55                  60

tta gaa tta ttg gaa agg gga gaa aga gaa aca cca atc ttt ctt gaa 240
Leu Glu Leu Leu Glu Arg Gly Glu Arg Glu Thr Pro Ile Phe Leu Glu
65                  70                  75                  80

tct ctg aca aga caa cta acg cag aaa ata tat gaa cta ata aaa aca 288
Ser Leu Thr Arg Gln Leu Thr Gln Lys Ile Tyr Glu Leu Ile Lys Thr
                85                  90                  95

aaa tct tta act gca gat gcc ttg caa caa ata agt gat aaa tac gat 336
Lys Ser Leu Thr Ala Asp Ala Leu Gln Gln Ile Ser Asp Lys Tyr Asp
            100                 105                 110

ggt gta gtg gca gaa aac aag ctg tta ttt ttg caa aga cag tat tat 384
Gly Val Val Ala Glu Asn Lys Leu Leu Phe Leu Gln Arg Gln Tyr Tyr
        115                 120                 125

gtt gat gat gaa gga aat gtt aga gat ggc cga aat aat gat aaa ata 432
Val Asp Asp Glu Gly Asn Val Arg Asp Gly Arg Asn Asn Asp Lys Ile
    130                 135                 140

tac tgt gag cca aag cat gta tac gac atg gtg atg gca aca cac ttg 480
Tyr Cys Glu Pro Lys His Val Tyr Asp Met Val Met Ala Thr His Leu

145                 150                 155                 160

atg aat aag cat ctt agg ggt aaa aca tta cat tcc ttt tta ttt tct    528
Met Asn Lys His Leu Arg Gly Lys Thr Leu His Ser Phe Leu Phe Ser
                165                 170                 175

cat ttt gcc aat att agt cat gcc atc atc gat tgg gtc cag caa ttt    576
His Phe Ala Asn Ile Ser His Ala Ile Ile Asp Trp Val Gln Gln Phe
            180                 185                 190

tgt tca aaa tgt aat aaa aag ggc aaa att aaa cca ttg aag gaa tat    624
Cys Ser Lys Cys Asn Lys Lys Gly Lys Ile Lys Pro Leu Lys Glu Tyr
        195                 200                 205

aaa cgt cct gac atg tac gat aaa cta cta cca atg gaa agg ata cat    672
Lys Arg Pro Asp Met Tyr Asp Lys Leu Leu Pro Met Glu Arg Ile His
    210                 215                 220

att gag gta ttc gaa ccc ttc aat gga gaa gct att gag gga aaa tat    720
Ile Glu Val Phe Glu Pro Phe Asn Gly Glu Ala Ile Glu Gly Lys Tyr
225                 230                 235                 240

tct tat gtc ctt tta tgc cga gac tat cgc tct agt ttt atg tgg tta    768
Ser Tyr Val Leu Leu Cys Arg Asp Tyr Arg Ser Ser Phe Met Trp Leu
                245                 250                 255

tta cca ctt aag agt acc aaa ttc aaa cat ctt atc cca gtt gtt tcc    816
Leu Pro Leu Lys Ser Thr Lys Phe Lys His Leu Ile Pro Val Val Ser

            260                 265                 270

tca ctt ttt tta aca ttt gct agg gtt cca att ttc gta aca tca agc 864
Ser Leu Phe Leu Thr Phe Ala Arg Val Pro Ile Phe Val Thr Ser Ser
        275                 280                 285

act tta gat aaa gat gat ctt tat gat att tgt gaa gaa att gca tca 912
Thr Leu Asp Lys Asp Asp Leu Tyr Asp Ile Cys Glu Glu Ile Ala Ser
    290                 295                 300

aaa tac ggt ctc cgt att ggc ttg ggt ttg aag agt tct gcg aga ttt 960
Lys Tyr Gly Leu Arg Ile Gly Leu Gly Leu Lys Ser Ser Ala Arg Phe
305                 310                 315                 320

cat act ggg ggt ata ctg tgc att cag tat gct cta aat agt tat aag 1008
His Thr Gly Gly Ile Leu Cys Ile Gln Tyr Ala Leu Asn Ser Tyr Lys
                325                 330                 335

aag gaa tgt cta gcc gat tgg ggt aag tgc cta aga tat ggc cct tac 1056
Lys Glu Cys Leu Ala Asp Trp Gly Lys Cys Leu Arg Tyr Gly Pro Tyr
            340                 345                 350

aga ttc aac cga agg aga aat aag aga acg aaa cgt aaa cct gtg caa 1104
Arg Phe Asn Arg Arg Arg Asn Lys Arg Thr Lys Arg Lys Pro Val Gln
        355                 360                 365

gta cta ctt agt gaa gtt cca ggt cac aat gcc aag ttt gag act aag 1152
Val Leu Leu Ser Glu Val Pro Gly His Asn Ala Lys Phe Glu Thr Lys

    370                 375                 380

aga gaa agg gtt ata gaa aac aca tat tcc cgt aat atg ttc aag atg 1200
Arg Glu Arg Val Ile Glu Asn Thr Tyr Ser Arg Asn Met Phe Lys Met
385                 390                 395                 400

gca ggt gga aaa ggt ctt ata tat ttg gaa gat gtc aat act ttt gcc 1248
Ala Gly Gly Lys Gly Leu Ile Tyr Leu Glu Asp Val Asn Thr Phe Ala
                405                 410                 415

ctt gct aat gaa gcg gat aat agc tgt aac aat aat gga att ctt cat 1296
Leu Ala Asn Glu Ala Asp Asn Ser Cys Asn Asn Asn Gly Ile Leu His
            420                 425                 430

aat aac aat ata gga aat gat aac ttt gaa gaa gaa gtg caa aaa caa 1344
Asn Asn Asn Ile Gly Asn Asp Asn Phe Glu Glu Glu Val Gln Lys Gln
        435                 440                 445

ttt gat cta act gaa aaa aac tat atc gat gag tat gat gat ttg gca 1392
Phe Asp Leu Thr Glu Lys Asn Tyr Ile Asp Glu Tyr Asp Asp Leu Ala
    450                 455                 460

cat gat tct tca gag ggc gaa ttt gaa cct aat acc tta act ccc gaa 1440
His Asp Ser Ser Glu Gly Glu Phe Glu Pro Asn Thr Leu Thr Pro Glu
465                 470                 475                 480

gaa aag cct cct cat aat gtc gat gag gac cga ata gag tcc acc ggc 1488
Glu Lys Pro Pro His Asn Val Asp Glu Asp Arg Ile Glu Ser Thr Gly

                485                 490                 495

gtg gca gcc cca atg cag gga aca gaa gag cct gaa aaa ggg gat caa 1536
Val Ala Ala Pro Met Gln Gly Thr Glu Glu Pro Glu Lys Gly Asp Gln
            500                 505                 510

aaa gaa agt gac ggt gca tca caa gta gat caa agt gtc gaa ata act 1584
Lys Glu Ser Asp Gly Ala Ser Gln Val Asp Gln Ser Val Glu Ile Thr
        515                 520                 525

aga cca gaa act tcc tac tat caa act ctg gaa tcg ccg tca aca aaa 1632
Arg Pro Glu Thr Ser Tyr Tyr Gln Thr Leu Glu Ser Pro Ser Thr Lys
    530                 535                 540

cga cag aaa tta gac caa cag ggt aat gga gat caa aca aga gac ttt 1680
Arg Gln Lys Leu Asp Gln Gln Gly Asn Gly Asp Gln Thr Arg Asp Phe
545                 550                 555                 560

ggc aca tca atg gaa ttg 1698
Gly Thr Ser Met Glu Leu
                565

<210> 28
<211> 927
<212> DNA
<213> Escherichia coli

<220>
<221> CDS

<222> (1)..(927)

<400> 28
atg gcg cgt tac gat ctc gta gac cga ctc aac act acc ttt cgc cag 48
atg gaa caa gag ctg gct ata ttt gcc gct cat ctt gag caa cac aag 96
cta ttg gtt gcc cgc gtg ttc tct ttg ccg gag gta aaa aaa gag gat 144
gag cat aat ccg ctt aat cgt att gag gta aaa caa cat ctc ggc aac 192
gac gcg cag tcg ctg gcg ttg cgt cat ttc cgc cat tta ttt att caa 240
caa cag tcc gaa aat cgc agc agc aag gcc gct gtc cgt ctg cct ggc 288
gtg ttg tgt tac cag gtc gat aac ctt tcg caa gca gcg ttg gtc agt 336
cat att cag cac atc aat aaa ctc aag acc acg ttc gag cat atc gtc 384
acg gtt gaa tca gaa ctc ccc acc gcg gca cgt ttt gaa tgg gtg cat 432
cgt cat ttg ccg ggg ctg atc acc ctt aat gct tac cgc acg ctc acc 480
gtt ctg cac gac ccc gcc act tta cgc ttt ggt tgg gct aat aaa cat 528
atc att aag aat tta cat cgt gat gaa gtc ctg gca cag ctg gaa aaa 576
agc ctg aaa tca cca cgc agt gtc gca ccg tgg acg cgc gag gag tgg 624
caa aga aaa ctg gag cga gag tat cag gat atc gct gcc ctg cca cag 672
aac gcg aag tta aaa atc aaa cgt ccg gtg aag gtg cag ccg att gcc 720
cgc gtc tgg tac aaa gga gat caa aaa caa gtc caa cac gcc tgc cct 768
aca cca ctg att gca ctg att aat cgg gat aat ggc gcg ggc gtg ccg 816
gac gtt ggt gag ttg tta aat tac gat gcc gac aat gtg cag cac cgt 864
tat aaa cct cag gcg cag ccg ctt cgt ttg atc att cca cgg ctg cac 912
ctg tat gtt gca gat 927